Europäisches Patentamt

European Patent Office

Office européen des brevets

(19)

(11) Publication number: **0 498 278 A1**

## (12) EUROPEAN PATENT APPLICATION

(21) Application number: **92101394.2**

(22) Date of filing: **27.01.92**

(51) Int. Cl.$^5$: **A01M 1/20**, A61L 9/03

(30) Priority: **29.01.91 GR 91010040**

(43) Date of publication of application:
**12.08.92 Bulletin 92/33**

(84) Designated Contracting States:
**AT BE CH DE DK ES FR GB GR IT LI LU MC NL PT SE**

(71) Applicant: **Lempidakis, Konstantinos E.**
**40-42 Megistis St. Kypseli**
**GR-11364 Athens (GR)**

(72) Inventor: **Lempidakis, Konstantinos E.**
**40-42 Megistis St. Kypseli**
**GR-11364 Athens (GR)**

(54) **Method and device for evaporating volatile substances contained in tablets.**

(57)    A new method and device which is based on the controlled evaporation and diffusion of substances, insect-repelling, aromatic, pharmaceutical or other use with tablets of special shape or on the evaporation and draining of substances impregnated in materials which are placed on an attachment with the shape of the special tablet, with a new method of heating/evaporation according to which, a special heat element (6) is vertically placed in the interior of a special device and enters in the interior of a special formed tablet (22) is internally and externally heated, while the created flowdynamics in the interior of the device contributes to the creation of a state of balance, adjusted from the current of natural convection, which enters the device assures a constant and controlled temperature and adjusts the evaporation speed.

Fig 1

EP 0 498 278 A1

Jouve, 18, rue Saint-Denis, 75001 PARIS

This invention is concerned with a new device and method which it is based on the controlled evaporation and the diffusion of substances of insect - repelling, aromatic, pharmaceutical or other use- ,from special shaped tablets or on the evaporation and draining of substances contained into materials which are placed on an attachment of a special tablet shape, with a new method of heating/evaporation,according to which a special heating element stands at the interior of a special formed talbet which covers it completely. This way two therodynamic equilibriums are created and the tablet is heated internally and externally, while the created flow dynamics in the interior of the device, contributes to the creation of an equilibrium state, adjusted from the natural convention flow which enters the device, assures a constant and controlled temperature and adjusts the evaporation speed. The special device design and the temperature gradient in this interior, assures the creation of an upward flow of air, normal and constant evaporation of the released substances and their diffusion into space. After it accumulates the quantities of heat required for the balance, a natural thermostatic condition is obtained and ends a steady state.

The up to date techniques usually offer evaporating devices for substances impregnated into tablets for insect - repelling or aromatic use. Their operation is not based on any special heating/evaporation method, which is required for a complet evaporation, with normal vapor flow, of the substance impregnated into material with heat insulation properties. On the contrary, these devices have a horizontal parallepiped electric heating place in the center of a natural air supplied box. Its size is 45 X 25 X 10 mm. An insect - repelling tablet of 35X23X2 mm is placed on it. The one side of the tablet is heated and, this heating process, causes evaporation of the impregnated substances into the surrounding space.

The standard tablets are consisted of heat insulating porous material, paper or cotton-paper and are impregnated with drastic insect - repelling or aromatic substances.

These devices and tablets produce a series of disadvantages:

(a) in order to operate, they require electric power of at least 5,5 - 8 Watts, while the energy required for the heating and the evaporation of the impregnated substances, is approximately 23 - 25% of that. The rest is wasted thus being impossible for them to operate with batteries (higher power consumption).

(b) the total heating pleace area is approximatelt 32 sq.cm while the tablet contact area is just 7,8 sq.cm. That means great waste of heat and need for more electric energy.

(c) the one side heating of the tablet and its construction material, which is heat insulated porous material, causes gradual and considerable temperature loss, from its contact area to the free one, resulting to a shifting (thermo-diffussing)of the impregnated substances gradually towards its interior where the temperature decreases, so there is no conveyor existing any more to transfer the heat from the heated contact area to the inside of the insulated tablet material. Therefore, it is necessary for the device in order to continue operating satis factory and limit the residue, to consume a lot more power and have higher heating temperature than this required normal evaporation of the substances. Also a kind of substances distillation appears at the inside of the tablet and an interstratification distribution according to their boiling points, so the emitted vapors have different form from the solution's, resulting to the reduction of the effectiveness when there is combined action.

(d) according to the above the tablet contact area is heated up to 180°C while the upper side reaches just 120°C, resulting to 50% fission of the substances before their evaporation (e.g. PAT. 38112 of " FUMAKILLA LTD ").

(e) the quantity of substances which fully evaporates is the one from the contact area, because of the tablet heating way and the design of the device. Therefore up to 10% of the impregnated substances remain in the tablet, in proportion to the tablet width and its material.

(f) the high and uncontrolled quantities of heat compared to the ones required by the tablet result to a vast evaporation during the first operating stages of the most part of the impregnated substances, so the quantity of the active ingredients remaining in the tablet for its further use, is too little.

(g) the heating place is positioned horizontally, though according to its shape it should be placed vertically (the smallest area downwards). Therefore, the heated air molecules which enter the device create, in their way out, swirling currents over the tablet which sweep off the substances, resulting to their condensation in the area around the heating place. So, after several uses, a quantity of oily substance seats around the heating apparatus which makes the device dangerous. CHADWICK and LORD, BULL.ENT. 67, 667-674 (1977) mention that the release degree of the drastic substances follows a negative exponential cuvre vs time.

So, the tablet effectiveness is limited to approximately 6 hours while for larger spaces is even more limited. The device can be dangerous, when used in limited spaces, due to the high concentration of the drastic substances.

To solve this problem,the use of additional substances which mix with the tablet drastics, has been proposed.

This solution is not satisfactory because, according to CHADWICK and LORD the percentage of the substances condensation increases the effectiveness decreases and the evaporation speed is affected to a small degree.

The present invention discloses a new method of controlled evaporation and diffusion of substances which are included into materials, a new insect-repelling, aromatic, pharmaceutical, draining and other uses device and a new type of tablet - attachment. In order to solve all the afore mentioned problems and to also accomplish applications which arise from its unique design. Its novelty consists of:

(a) The method of controlled evaporation which consists of the creation of two thermodynamic equilibriums with heat exchange, (crating flux from the source to the perimeter and reverse) which develop inside a vertical space of perimetric surface, with double walls, the interior of which, consists of insulating meterial, with insulation between them, with the top and bottom end open. A heat source, located in the center of the space, in top or around of which the impregnated material is seated and by the accumulation of the released heat to a zone thermodynamically calculated and constructed, around and across the heat source in the interior of the thermo - insulated area. The heat accumulation can be boosted with direct transfer of thermal power from the source to the previously mentioned zone. These equilibriums are forced to reach a thermodynamic balance having as an adjusting factor the flow - dynamic condition,that is created from the vertical current of natural convection, that enters into the area through a variable diaphragm and is self-adjusted by the temperature difference between the hot area and the rest space under it.

(b) A new insect-repelling, aromatic, pharmaceutical, draining and of other uses device,with a special tablet-attachement, is a indicative application of the above method. The method is applied with the creation of the thermodynamic equilibriums (from which arise conjugated and opposite flux) into the device interior . The equilibriums reach a thermodynamic balance, due to the design of the device, for every desirable evaporation speed, which control, while the developed flow - dynamics in the interior of the device, are adjusted from the current of natural convenction which enters and secures constant and controlled temperature/evaporation and diffusion speed adjustement into space. With the special design of the device, the creation of upward air current inside it and the sweep of the released vapors instantly is obtained with upward flow of convection natural without them being allowed to remain on it. Also innovation is the placement of the heat element verically, which facilitates the natural convenctions of upward air current without the creation of swirling currents. Also the tablet is both, internally and externally heated. Finally, the novelty consists of the way of the alternative adaptation of the heat element thus being possible for an alternative operation of the device with standard tablets.

(c) A specially designed, according to the use tablet - attachment with a split-housing, with shape and size corresponding to the heat element so the tablet enters its interior, covers it completely and internally heats it. The tablet can, according to the use, be made of paper or cottonpaper or other material. Its properties should harmonize with the parameters of the heating process. According to its alternative design, it could be either impregnated with several substances or two cases could be added to its exterior surfaces, which replace part of its thickness and to which the material for draining or drastics release is seated. In this case the tablet is not to be disposed but it can be used as an attachment for multiple uses and its manufactured material and technical characteristics should harmonize with the physical properties of the material which is to be evaporated or drained.

Also another innovation, is the new way of heating the tablet-attachment which is obtained by its apropriate design, in such way as the heat element to heat it internally and so to create a thermodynamic equilibrium which adjustes the mass and heat transfer from the inside of the tablet to the outside.

The advantages offered by the invention are:

(a) With the new method a thermodynamic system is obtained which changes its entropy by itself according to the supply change of the entering air, creating finally a steady state in every stage, without the change of the quantity of heat supplied. With this method the convertion of a constant continuously released thermal energy of quantitative form to equivalent qualitative and reverse is obtained,without the respective change of the supplied thermal power. Also, due to the obtained physical thermostatic state, quantities of heat of required and constant temperature are released respectively in every operation stage. This temperature is the desirable one and remains constant to every operation stage.

(b) This new device is achieved reduction of the electric power and energy save, uniform distribution of heat at the tablet mass so as no added substances are used for the improvement of the drastics flow, the fission of the substances before their exit from the tablet is avoided, the action time is increased and the effectiveness is prolonged because of the drastics constant flow into greater period of time. The condensation of substance is avoided and the best diffusion is obtained. Also, two tabltes can be used simultaneously into this device e.g. one insect-repelling and one aromatic, depending, on the desirable use. Finally, by this device we can obtain uniformed controlled evaporation and material draining, at adjusting temperatures, without any control systems, without decomposition of sensitive to heat substances contained into the materials, without any remaining humidity. A significant advantage of the device is that it can operate either with electric current or with battery, because of the least consumption of electrics energy.

(c) The new type talbet-attachement, which is heated on all its surfaces, obtains uniformed evaporation, solving the most of the aforementioned problems of the standard tablets. Also, because of its alternative (dependingon the use) shape and its special design (when needed) - the outside side housings - it can be used either as an one use tablet or as a multiple use attachement for draining of materials, which are placed in any form in its housing - cases or for the evaporation of substances which many have aromatic, pharmaceutical, therapeutical, insect-repelling or other use.

A detailed description of the new method, the new device, which is its indicative application and the new type tablet-attachment, is given below, with reference to drawings which explain their operation and the materialization of an indicative example.

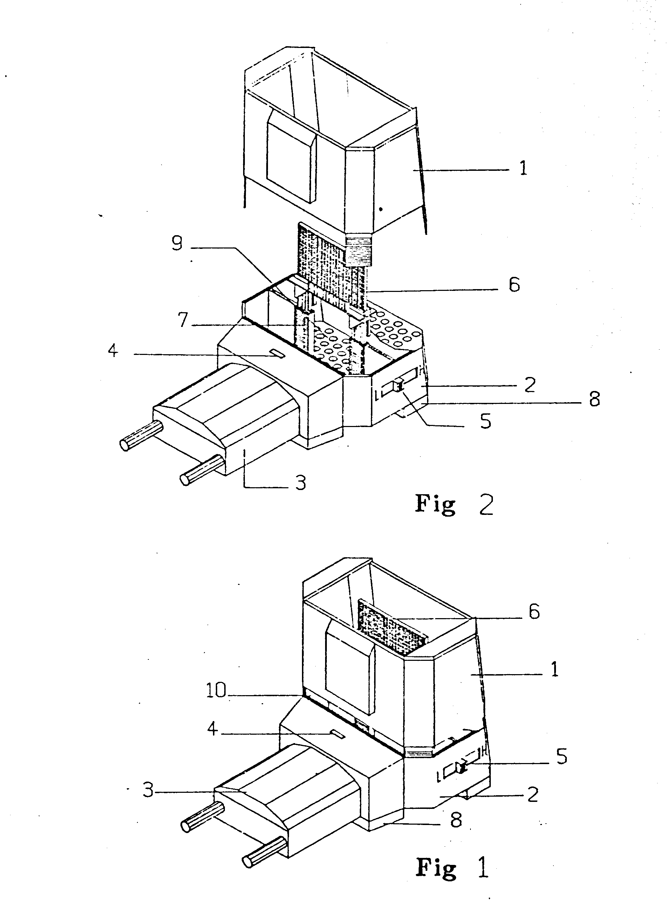

The figure 1 shows a general view of the device according to the invention, assembled without tablet, in order to be used with electric current 220V, without lead.

The figure 2 shows a view of the same device with open cover, without tablet, where the heat element is shown.

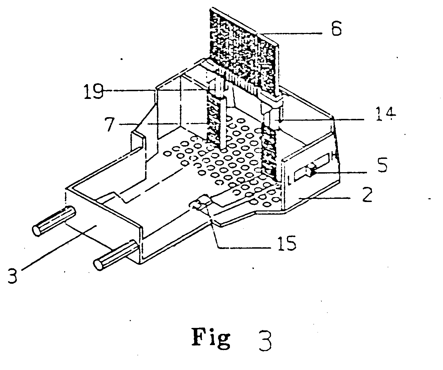

The figure 3 shows a cross section of the same device without cover and tablet, so the conjunction of the circuit with the heat element is shown, without the position of the electric resistance being indicative.

The figure 4 shows the device with open cover, without tablet but with lead and supply entrance of battery. It can also operate alternatively with current 220V or battery.

The figure 5 shows the device of figure 4 assembled (with closed cover).

The figure 6 shows the heat element with support plate of the tablet.

The figure 7 shows the heat element enlarged in more detail.

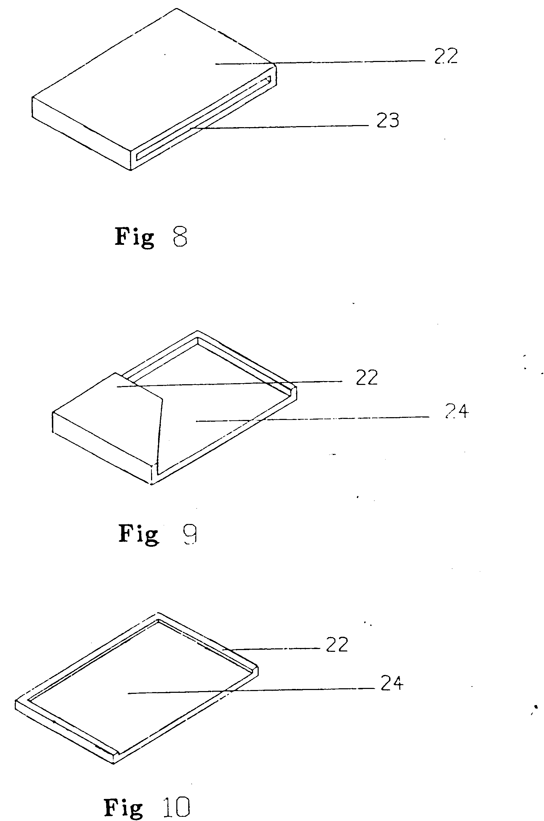

The figures 8, 9 and 10 feature in the tablet exterior where the split is shown to which the heat element enters (figure 8) the cross-section,where the inside wall is shown (figure 9) and the complete section (figure 10).

The figures 11 and 12 show variation of the heat element in case of using the device with standard tablets.

The figure 13 shows an alternative shape of the heat element for exclusive use of the device with standard tablets.

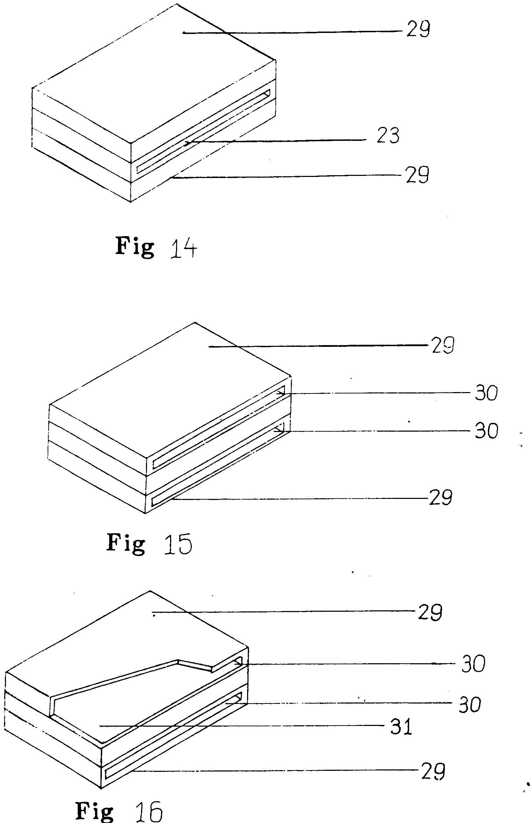

The figures 14, 15 and 16 show a perspective alternative form of a new tablet, which according to the invention, it is used as attachment for multiple alternative uses, for draining of materials for evaporation of substance of any kind and form.

All figures, referring to the above invention and perspective and without scale.

According to the invention, the new methods of controlled evaporation and diffusion of substance impregnated into materials, consists of the creation of two thermodynamic equilibriums inside a insulated thermodynamic system, from which derive equivalent conjugated flux of opposite direction (through which the heat exhange between the equilibrium is happening), which are forced to balance through the created thermodynamic yrocess, having as adjusting factor the flow - dynamic condition created from a current of natural convenction and it is self - adjusted from the temperature difference between the created hot area and the space under it.

These equilibriums are developped inside a, thermodynamically calculated and thermoinsulated closed perimetrically space, which delimit from a standing perimetric and externally thermoinsulated surface,which can have an area reduced with height (e.g. chimney), where its toy and horizontal end is open and there is a diaphragm at the bottom end which can be variable and which can be calculated, so that the volume of the required air enters according to the desirable temperature level or two parallel diaphragms from which one is steading for the max supply and one is variable for the minimum.

The first equilibrium is a result of the heat supply, from the source to the system and is created by the heat exchange from the source to the perimeter and reverse.

In the interior of the thermoinsulated perimetric area, across and around the heat source, a hot area-zone is created from the accumulation and the heat exchange, which is released from the heat sources (second equilibrium).

In the interior of the thermoinsulated perimetric surface attached to it and exactly across the heat element, an appropriate calculated and manufactured perimetric surface-zone can be placed. Its size should be the same with the mentioned hot area - zone. This surface may have a heat source in its interior connected in series with the heat source at the area center, which consists of material with appropriate heat capacity, coefficient of heat conductivity and emission and abosorption of the heat radiation when a boost of the accumulation and exchange of the necessary quantities of heat transfer, are required.

The system, through the thermodynamic processes of equilibrium which take place in its interior, obtains a thermostatic state compensating the heat flux after thermodynamic stability has been created. During the first stages flux are created from the perimeter towards the center and reverse, the total energy of the systemmaterial initially changes and by creating heat accumulation, and after compensating the flux, the equilibrium is obtained. The system goes to thermodynamic stability and releases constant quantities of mass and energy to the outside, while its internal energy remains constant.

When thermodynamic instability is imposed to the system with the increase of the supply of the entering air, the system shows a new state which is stabilized again with the accumulation of the respective heat quantities after a short period of time and reaches a new steady state.

Some changes take place when the instability is caused from an internal factor of the system (e.g. change of heat consumed into the system as evaporization enthalpy, change of tablet heat demands) disturbing the steady state.

During the transition stage to another steady stage, the system falls to an unsteady thermodynamic state and then through the flux compensation and the adjusting factor, which is the creating flow-dynamics, goes to a new steady stage.

The above figures show and indicative application on the invention, that is the application of the method of controlled evaporation and diffusion of substances into a new insect-repelling, aromatic, pharmaceutical, draining and other uses, device with a special tablet - attachment to which the method is applied by the creation of the thermodynamic equilibriums (from which derive conjugated and opposite flux) in the interior of the devise, which balances thermodynamically (because of its design) in every desirable evaporating speed, while the flowdynamic being developed in the device and adjusted from the current of natural convenction, obtains constant and controlled temperature,adjustment of the evaporating speed and diffusion of substances into the surroundings.

With this special design we obtain the creation of an upward air current and the released vapors is drifted instantly with natural flow upwards without any remaining residue. This new device consists of a thermoinsulated cover (1) trapezoid, tapered or any other similar shape (chimney), of a base (2), of the air inlet openings at the cover (10) and at the base (9), of a heat element (6), the shanks (7), for the heat element housing and its supply with current, of the current receiver (3) or electric lead (12) for 220V current,of the inlet for alternative battery supply (13), of the operation lamp indicator (4), of the switch (5) for the adjustment of release and evaporation of the substances in two scales. Two legs (8) support the device in order to stay higher than the surface which is placed, so creation of air current from the holes under (9) is obtained. Also it can be held by hook (11).

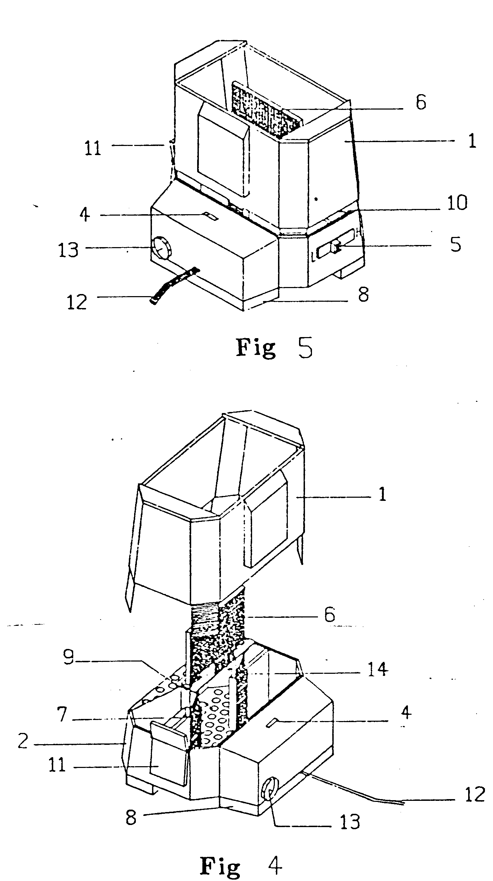

According to the present invention, the heat element (6) is a flat surface made of ceramic or heat resistant insulating material with thickness, heat capacity and other technical characteristics which agree with parameters of the acting processes, placed at the center and vertically on the base (2) and under the opening of the cover taper of the device. The interior of the heat element on figure 5 consists of 3 layers and particularly: at the center there is a thin plate which is the thermal resistance for the battery current. This plate is covered in both sides by the resistance surfaces of 220V current. Heat factor can be, besides the resistance, any material which heats up when current goes through it. For the alternative use of the device with electric current or battery, the four resistance end connections lead up to two each leg surface (14) and through the conductive zones (19) they are connected with the respective shanks (7) for the housing of the heat element. Each shank (7) is connected with the current receiver (3) for 220V current supply or with electric wire (12) and the housing (13) for supply from battery. The device can be supplied with 220V current while the heat element requires less voltage (e.g. 110V) with the connection of the appropriate resistance elements in series.

The ring (20) obtains the required distance of the heat element (6) from the support plate (10) in order to create the gap (21). The housings (18) support the plate (16) which is attached to the upper shanks surface (7) during the placement of the heat element into the shanks housings.

By entering the heat element into the tablet interior (perfect fit) a thermodynamic equilibrium is created (first equilibrium) and together with the contribution of the second equilibrium and the created flow-dynamic condition, the heat control and therefore the evaporation control, is obtained. The heat element must have the same temperature throughout its surface and should continuously produce constant heat quantities to every operation scale. These heat quantities correspond to the quantities required from the tablet for the normal and complete substance evaporation.

The device cover (1) is a vertical perimetric trapezoid surface, with the top and bottom horizontal end open, with double walls and insulation between them, so there is no heat escape, with variable position on the device and during its placement four side openings (10) are formed which change according to its position. In the interior and at the area across the heat source, an appropriately calculated and manufactured perimetric surface can be placed, when it is required a boost of the accumulation and exchange of the necessary heat quantities or a transfer of thermal power quantities from the heat element, through an appropriate resistance connected in series.

This surface should have the appropriate technical specifications matching with the parameters of the thermodynamic processes which take place.

The second equilibrium is created in the cover interior across and around the heat element where the hat area-zone is forming by the heat accumulation and the exchange which is released from the heat element.

By the current supply to the device the heat is released from the heat element, which is transferred to the tablet, the temperature gradient is increased and the process of flux creation and flow dynamics starts.

Some of the heat quantity is getting out from the device, while another greater amount is accumulated into its interior until the device reaches equilibrium and the accumulation is eliminated. Then the heat supply is used for the evaporation process and for the maintenance of the transfer phenomena in its interior.

With the vertical position of the heat element and the air inlet openings (9 and 10) at the four cover sides (1) and at the bottom surface base (2) and because of the maintained gradient temperature, the creation of a constant vertical upward air current throughout the devices obtained, which sweeps upwards the evaporating molecules of the substances without change of the flow direction, because of striking against an obstacle (natural vapor attraction of the tablet).

By increasing or decreasing the air flow by removing the cover (1), the device can be adjusted into permanent constant desirable levels of temperature that we select. The vertical placement of the heat element is in accordance to its shape (parallelogram). That means that if the invention is applied with a heat element of a different shape,then the correct placement should be different, in order to facilitate the flow-dynamics in the interior of the device.

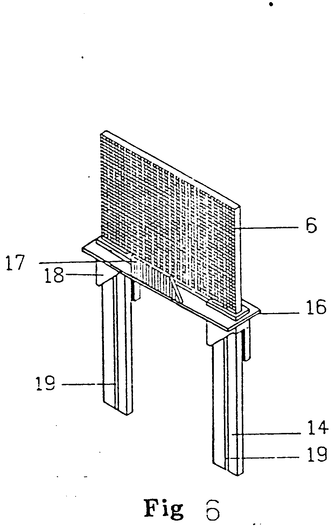

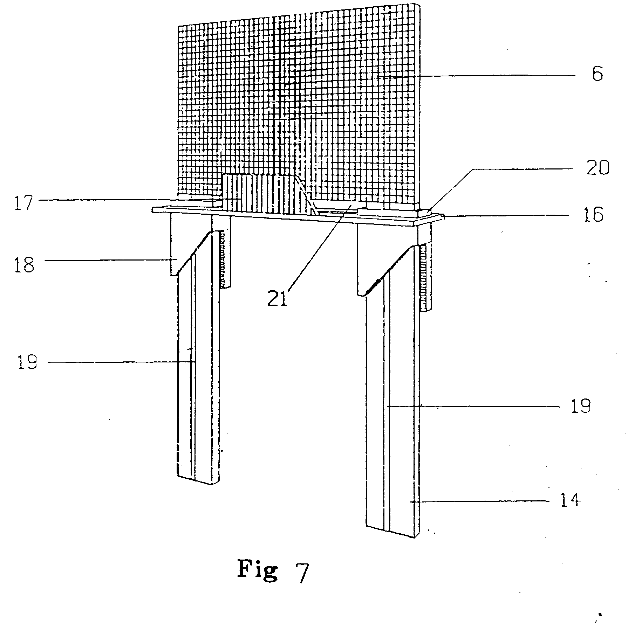

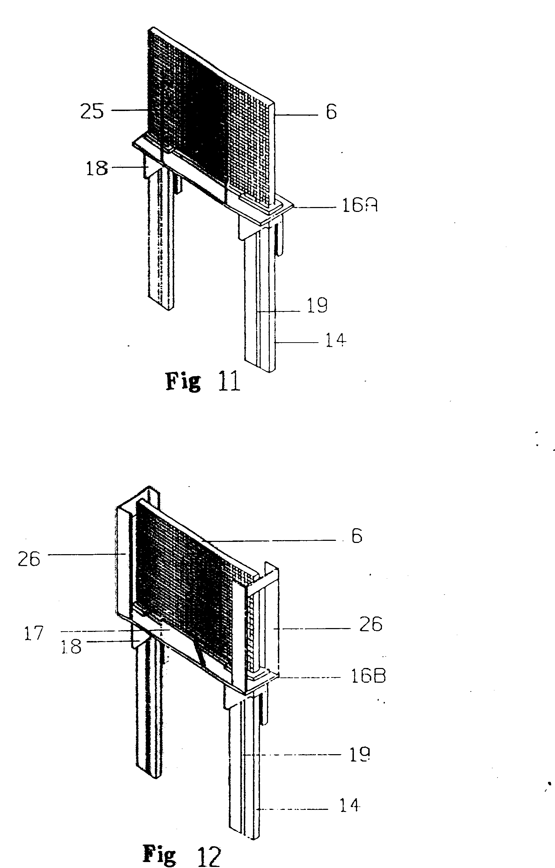

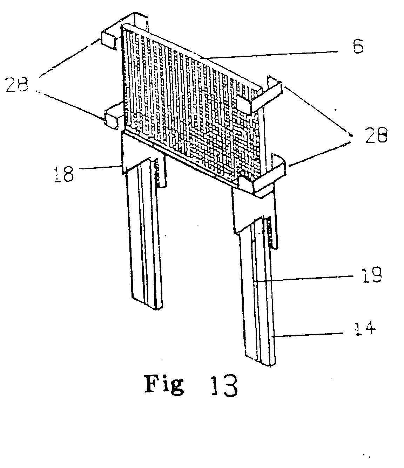

The device can be manufactured in such way as to be able to operate either with battery only or with electric current, therefore the thermal element includes only one resistance with its lead ends ending to two conductive zones (19), each one in every leg (14). Same change takes place in the interior of the two shanks (7) and the device has only one current receiver (3). According to the invention, the device can operate, when desired, with the standard tablets, without losing its advantages. In this case either the heat element is replaced (figures 6 and 7) with another (figure 13) or a respective plate with the sheets is placed on it (figure 11 and 12).

The figure (11) shows a variation of the heat element of figures 6 and 7, in which the support shank (17) is missing on the support plate of the tablet (16). Instead of that, there is a wire sheet (25) on the plate (16A) from both sides of the heat element for holding and the fit of the standard tablets.

The figure 12 shows another alternative heat element, in case of standard tablet use, which differs from the standard one (6). This difference consists of two housing-cases fixed at both ends on the plate (16B), as this is shown in figures 6 and 7. They hold the standard tablets and obtain the perfect fit with the heat element with the clip (17).

The figure (13) shows an additional alternative form of the heat element for the case of possible exclusive use of the device with standard tablets. Here the plate (16) is completely omitted while the standard tablets hold from the four support sheets (28), two in each end or just two diagonally for their perfect fit with the heat element.

The complete and effective operation of the device is obtained by the new type tablet (8) with the thin split-housing (23), of the same size as the heat element, which enters it entirely in order to heat its interior (24).

The tablet is also heated up on its both outside surfaces from the heat which is accumulated at the device cover. The device operates consuming the least electric, a fact which permits the alternative operation with battery.

The tablet can be made of paper, cotton paper or anything else (it depends on its use). The properties and the technical characteristics of the material should harmonize with the parameters of the heating process.

Also, due to the tablet's alternative form as shown in figure (14), it can be used for material draining or substances release with the addition of the two housings (29) to the outside surfaces. The material for draining or evaporation, is placed on the two outside housings of the the tablet-attachment. In this case the tablet is not deposed after the use, but it is used as an attachment. Its manufactured material should have thermal properties which harmonize with the respective physical properties of the placed material.

The new method of controlled evaporation and diffusion of substances impregnated into materials, the new device and the special tablet-attachment can apply for material draining, besides the insect-repelling, pharmaceutical, aromatic, therapeutical or other uses.

In case all above uses require certain condition (e.g. temperature, diffusion of substances etc) the method, the device and the tablet have the ability to adjust.

The method and the device can be also used as follows:

a) for household use and for the manufacturing of heating devices which will supply regulating temperature and humidity to the surroundings,with the least consumption of electric power.

b) in industry, when a supply of constant heat quantities of thermostatic temperature is required.

c) as a part of devices which require heat supply with the above specifications.

d) in any case we need to obtain heat supply without temperature decrease in the interior of materials and of the thermoinsulated materials for several applications.

## Claims

1. A new method for the achievement of evaporation and diffusion of substances impregnated into materials for insect-repelling, aromatic, pharmaceutical. therapeutical, draining or other uses, which consists of the creation of two thermodynamic equilibriums inside a vertical, thermoinsulated perimentric area, with open the top and bottom ends, which are forced to balance thermodynamically through the flowdynamics developed from the vertical-current of natural convenction, entering from the area's bottom end, characterised by the fact that these equilibriums develop in a thermodynamically calculated and thermoinsulated space, which is perimetrically closed and delimits from a vertical perimetric and externally thermoinsulated surface which can have a area reduced with height (e.g. chimney), where its top and bottom horizontal end is open and there is a diaphragm at the bottom end which can be variable and which can be calculated so the required volume of air enters according to the standard temperature levels needed or two parallel diafragms from which one is steady for the max supply and one is variable for the minimum. The first equilibrium is a result of the heat supply by the source or the source and the material placed in it to the system and is created from the heat exchange form the source to the perimeter and reverse. In the interior of the thermoinsulated perimetric area, across and around the heat source, a hot area-zone from the accumulation and heat exchange which is released from the heat source is created (second equilibrium).

When a boost of the accumulation and the exchange of the necessary heat quantities is required an apropriate calculated and manufactured perimetric surface can be placed in the interior of the thermoisulated perimetric surface, attached to it and exactly across the heat element, of the same size with the hot area-zone, which can have a heat source in its interior connected in series with the heat source at the center of the area. The zone consists of material with apropriate heat capacity, coefficient of heat conductivity and emission absorption coefficient of the heat radiation.

The system, trough the thermodynamic processes of equilibrium which take place in the interior, obtains thermostatic state with the compensation of the heat flux and after thermodynamic stability has been created.

During the first stages flux are created from the perimeter towards the center and reverse, the total energy of the system source-material initially changes and then by the creating heat accumulation obtains compensation of flux and equilibrium, the system goes into thermodynamic stability and releases constant quantities of mass and energy to the outside, while its internal energy remains constant.

When thermodynamic instability is imposed to the system with the increase of the sypply of the entering air, the system shows a new state which is stabilized again with the accumulation of respective heat quantities after a short period of time and reaches a new steady state.

During the transition stage to another steady state, the system falls to an unsteady thermodynamic state and then through the process of flux compensation and the adjusting factor which is the creatad flow-dynamics, goes to a new steady state.

By this method it is obtained a transformation of a continously constant released thermal energy of quantitative form,to an equivalent qualitative and reverse, without the respective change of the supplied electrical power. Also, due to the obtained thermostatic, quantities of desireable and constant temperature are released, which correspond to every stage of operation.

Thus, the method assures constant and controlled evaporation and diffusion of the impregnated substances, consuming the least thermal power.

2. A heat element (6) characterised a flat surface made of ceramic or heat resistant insulating material with thichness, heat capacity and other technical characteristics which agree with the parameters of the acting processes, placed at he center and vertically or the base (2) and under the opening of the cover taper of the device.

The interior of the heat element consists of 3 layers and particularly: at the center there is a thin plate which is the thermal resistance for the battery current.

This plate is covered on both sides by the surfaces of the resistance for 220 V current. Heat factor can be, besides the resistance, any material which heats-up when current goes through it. For the alternative use of the device with electric current or battery, the four resistance end connections lead up to two in each leg surface (14) and through the conductive zones (19) they are connected with the respective shanks (7) for the housing of the heat element.

Each shank (7) is connected with the current receiver (3) for 220 V current supply or with electric lead (12) and with the housing (13) for wupply form battery.

The device can be supplied with 220 V current while the heat element requires less voltage e.g. 110 V with the connection of the apropriate resistance elements in series. According to the above alternative design of the heat element, the device can operate either with 220 V current or with battery.

3. A heat element (6), according to the claims 2 and 3, which is intended for the operation of the device only with 220 V current, characterides by the fact that it consists of a heat plate according to figure 6 or 7, it has the proportionate connection in a way that the resistance terminals end each, to each conductive zone (19) at the legs (14).

4. A heat element (6) according to claims 2 and 3 which is (characterized in that is) intended for the operation of the device only with battery and is characterised in it consists of a heat according figures 6 and 7, with that tesistance of apropriate elements for the operation of battery current and it has two terminals which end to another leg (14).

5. A heat element, which differs form the heat element (6) of claims 1, 2, 3, 4, 5, of figures 6 and 7 characterised in that:

a) a variation of a support plate (16A) is constructed, instead of the support plate (16) which is assembled on the heat element (6) and to which there is a wire sheet (25), instead of a clip (17), as a connector from both sides, so a vertical standard tablet can be placed between it and the heat element, each from each side (figure 11) or:

b) a variation of a support plate (16B) is constructed instead of the support plate (16) as figure 12 shows, where in addition to drawings 6 and 7 and claims 2 and 3, there are two housings (26) fixed in both ends which hold the standard tablets and secure, with the clip (17), the perfect fit with the heat element or:

c) a variation of a heat element is constricted for alternative use of the device, exclusively with standard tablet, where the plate (16) is completely omitted, while four support sheets(28) hold the standard tablet, two in each end or just two diagonally, for their perfect fit with the heat element.

All three of the above alternative forms of the heat element, as well as the heat element (6) according to claims 2 and 3, enter and exit easily from the device with the help of a current receiver - socket between the device support shanks (7) and the legs (14) of the heat element, while the housing (18) secures the gap (21) which protects from unecessary heat loss.

6. A new insect-repelling, aromatic, therapeutical, pharmaceutical, draining and other uses, device with a special tablet -attachment, which is an indicative application of the above method, to which the method applies by the creation of the thermodynamic equilibriums (from which derive conjugated and opposite flux) in the interior of the device, which balance thermodynamically (because of its design) for every desireable evaporating speed which they also control, while the flowdynamics, developed in the device interior and adjusted by the current of natural convention, obtains constant and controlled temperature, adjustment of the evaporating speed and diffusion of substances into the surrounding area.

The device is specially designed in order to achieve the creation of an upward air current and the released vapor is drifted instantly with natural convection upwards without any remaining residue.

This new device is characterised in that it consists of a thermoinsulated cover (1) trapezoid, tapered or any other similar shape (chimney), of a base (2), of the air inlet openings at the cover (10) and at the base (9), of a heat element (6) with its alternative forms, the shanks (7) for the heat element housing and its supply with current, of the current receiver (3) or electric lead (12) for 220V current, of the inlet for alternative battery supply (13), of the operation lamp indicator (4), of the switch (5) for the adjustment of release and evaporation of the substance in two scales.

The device is supported by two legs (8) in order to stay higher from the surface on which it is placed, so as creation of air current from the holes under (9) is obtained. It can also be held by a hook (11).

The proper and effective operation of the device is obtained by the new type tablet (8) with the thin split-housing (23), of the same size as the heat element, wich enters in it entirely in order to heat its interior (24). This tablet is heated on its both outside surfaces from the heat which is accumulated at the device cover. The device operates consuming the least electric power, a fact which permits operation also with battery.

The device can also operate with an attachment which has a thin split-housing (23), same as the tablet, of same size as the heat element (which enters entirely in it in order to heat it in its interior) and has two housing-cases (29) at the the exterior surfaces, when the device is to be used for material draining or release of drastic vapors placed in the housing cases. This attachment is a functional part of the device. The device cover (1) is characterized in that it is a vertical perimetric trapezoid surface, with the top and bottom horizontal end open, with double walls and insulation between them so there is no heat escape, with variable position on the device and during its placement four side openings (10) are formed which change according to its position.

In the interior and at the the area across the heat source, an appropriately calculated and manufactured perimetric surface can be placed, when it is required a boost of the accumulation and exchange of the necessary heat quantities or transfer of thermal power quantities from the heat element,through an appropriate resistance in series. This surface should have the appropriate technical specifications in order to match with the parameters of the thermodynamic processes which take place

7. A new type tablet, according claim 6, characterized in that it has a thin split-housing (23) of the same size with the heat element which enters entirely in it in order to heat it in its interior (24). This table is also heated on its exterior surfaces from the heat which is accumulated at the device cover.

   The tablet can be made of paper, cotton paper or of the same material of which is drastic substances should be evaporated. (All depends on its use), the properties and technical specifications of which should harmonize with the parameters of the heating process.

8. An attachment of tablet-shape, according to the claims 6 and 7 characterized by a thin split-housing (23) of the same size as the heat element, which enters entirely in it in order to heat it in its interior and has outside housings (29), in which the material for draining or drastics release is placed which (outside housings) can be made of porous or other material (it depends on the use) and their thermal properties must harmonize with the physical properties of the material placed in them and the parameters of the heat process.

9. The use of the method and the device, according to the claims 1, 2, 3, 4, 5, 6, 7 and 8 for insect-repelling, aromatic, pharmaceutical, therapeutical draining and heating use, without being limited by these uses.

   The use of the method and the device according the claims 1, 2, 3, 4, 5, 6, 7, 8 and 9:

       a) for household use and for the manufacturing of heating devices which will supply regulating temperature and humidity to surroundings with the least consumption of electric power.

       b) in industry, when a supply of constant heat quantities of thermostatic temperature is required.

    c) as a part of devices which require heat supply with the above spefications.

    d) in any case it is required to obtain heat supply without temperature decrease in the interior of materials and of the thermoinsulated materials for several applications.

10. The alternative use of the device with standard tablet with a variation of the support plate according the claim 5 (a) and (b) or of the heat element according the claim 5 (c).

    As well as with the attachment according to the claim 8 for material draining or evaporation of drastic substances impregnated in it or evaporation of material or substances which are placed in its housings.

Fig 2

Fig 1

Fig 3

Fig 5

Fig 4

Fig 6

**Fig** 7

Fig 8

Fig 9

Fig 10

**Fig 11**

**Fig 12**

Fig 13

Fig 14

Fig 15

Fig 16

EP 0 498 278 A1

<table>
<tr><td colspan="2"></td><td>EUROPEAN SEARCH REPORT</td><td colspan="2">Application Number</td></tr>
<tr><td colspan="2">European Patent Office</td><td></td><td colspan="2">EP 92 10 1394</td></tr>
</table>

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | GB-A-2 173 103 (PFIZER INC.) <br> * page 1, line 64 - line 70; figures * | 1-6,9,10 | A01M1/20 <br> A61L9/03 |
| X | EP-A-0 198 373 (HENKEL KOMMANDITGESELLSCHAFT AUF AKTIEN) <br> * column 4, line 19 - line 51; figures 1,3 * | 1 | |
| X | FR-A-2 378 448 (MATSUSHITA ELECTRIC WORKS LTD ET AL.) <br> * claim 6; figures 3-5 * | 1 | |
| A | GB-A-2 057 884 (EARTH CHEMICAL COMPANY LIMITED) <br> * page 6, line 36 - line 51; figure 12 * | 7,8 | |
| A | US-A-4 037 352 (C. HENNART ET AL.) <br> * column 8, line 37 - line 54; figure 2 * | 7,8 | |
| A | FR-A-2 477 835 (GLOBOL-WERK GMBH) | | |
| A | US-A-2 931 880 (D.W. YAFFE) | | TECHNICAL FIELDS SEARCHED (Int. Cl.5) |
| A,D | BULL. ENT. RES. <br> no. 67, 1977, <br> pages 667 - 674; <br> P.R. CHADWICK ET AL.: 'TESTS OF PYRETHROID VAPORISING MATS AGAINST AEDES AEGYPTI (L.) (DIPTERA: CULICIDAE)' | | A01M <br> A61L |
| A | FR-A-2 054 435 (FUMAKILLA LIMITED) | | |

The present search report has been drawn up for all claims

| Place of search | Date of completion of the search | Examiner |
|---|---|---|
| THE HAGUE | 07 MAY 1992 | MARANGONI G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

19